# DEMANDE DE BREVET EUROPEEN

(11) **EP 2 011 470 A1**
(43) Date de publication de la demande: **07.01.2009**
(21) Numéro de dépôt: 08159498.8
(22) Date de dépôt: 02.07.2008
(51) Int. Cl.: A61K 8/02, A61K 8/73, A61K 8/99, A61Q 19/08, A61Q 19/02

(54) **Ensemble comportant un substrat comportant de la biocellulose et une composition cosmétique pulvérulente à mettre en contact avec le substrat**

(30) Priorité: 02.07.2007 FR 0756216
(71) Demandeur: L'Oreal, 75008 Paris (FR)
(72) Inventeur: Legendre, Jean-Yves, 75015, Paris (FR)
(74) Mandataire: Tanty, François

(57) **Abrégé**

La présente invention concerne un ensemble (1) comportant :
- un substrat comportant de la biocellulose,
- une composition cosmétique pulvérulente au moins partiellement hydrosoluble à mettre en contact avec le substrat.

## Description

La présente invention concerne les procédés de préparation d'une composition cosmétique et les ensembles permettant de préparer et d'appliquer une composition cosmétique.

Il existe dans certains cas un besoin pour augmenter le temps de contact entre un site d'application et une composition cosmétique afin d'en optimiser l'efficacité.

Pour cela, différents substrats ont été développés dans le but d'être imprégnés d'une composition puis d'être appliqués sur le site à traiter. La composition ainsi imprégnée dans le substrat diffuse plus longtemps vers le site d'action, comparativement à la composition sans substrat. On connaît ainsi par les publications EP1095589, EP1338264, US6702792 ou WO 06053332 des masques de soin visage en substrat non-tissé imprégnés de compositions cosmétiques de type émulsion pour des actions blanchissantes ou anti-âge.

Des produits similaires pour des applications corps amincissantes sont également apparus récemment sur le marché (Bandes anti-cellulite, Body Sculptess, Lancôme).

D'autres substrats tels que des gels de polysaccharides naturels, peuvent également être employés, comme divulgué dans la publication US 2003/0113356, pour réaliser des articles imprégnés.

Bien que pratiques d'utilisation, ces articles cosmétiques peuvent présenter l'inconvénient de contenir des concentrations relativement élevées en conservateurs. En effet, les différents substrats employés sont prompts à la contamination microbienne.

En outre, leur fabrication requiert une étape d'imprégnation de la composition cosmétique sur le substrat. Cette imprégnation est parfois difficile à réaliser de façon homogène et surtout peut évoluer au cours du temps et conduire à une sédimentation de la formule cosmétique.

On connaît également des publications US 2001/0028894, US 6730317 et WO 03/075820 d'autres systèmes d'administration prolongée de substances cosmétiques tels que des patchs. Dans ce cas, des ingrédients actifs sont directement incorporés dans la matrice du patch qui est ensuite placé sur le site d'action.

Le choix de la matrice conditionne l'adhésivité du patch. Le recours à des polymères chimiquement réticulés tels que des polymères acryliques peut provoquer des réactions non souhaitées sur la peau et ne permet pas d'obtenir des articles écologiquement responsables.

En outre, les inconvénients majeurs de ces systèmes sont leur difficulté à les formuler avec tous types d'actifs et à hautes concentrations, ainsi que la faible diffusion d'actifs vers le site d'action du fait de la cinétique relativement lente.

Enfin, ces patchs offrent peu de sensations cosmétiques et aucun bénéfice immédiat ne peut être obtenu.

L'invention vise à proposer une nouvelle façon de préparer et/ou d'appliquer une composition cosmétique, remédiant à tout ou partie des inconvénients ci-dessus.

Selon l'un de ses aspects, l'invention a pour objet un ensemble comportant :
- un substrat comportant de la biocellulose,
- une composition cosmétique pulvérulente au moins partiellement hydrosoluble à mettre en contact avec le substrat.

L'ensemble ci-dessus peut être proposé au consommateur dans un même emballage, par exemple boîte, blister, coffret, sachet ou récipient à compartiments multiples.

Le substrat peut être humide ou en variante être sec et destiné à être réhydraté, notamment extemporanément. L'ensemble peut alors comporter une solution aqueuse pour réhydrater le substrat. En variante, le substrat est réhydraté avec de l'eau.

La solution aqueuse peut éventuellement comporter au moins un actif incompatible avec au moins un actif contenu dans la composition.

La solution aqueuse peut comporter une eau minérale, thermale ou une eau de plancton, entre autres. Dans le cas d'une eau de plancton, la composition cosmétique pulvérulente peut contenir le plancton, par exemple sous forme lyophilisée.

La composition cosmétique pulvérulente peut avoir une granulométrie moyenne allant de 5 µm à 1000 µm, voire allant de 50 µm à 500 µm

La composition cosmétique pulvérulente peut contenir au plus 10 % en masse d'eau, voire au plus 5 % en masse d'eau, voire être complètement anhydre.

L'invention permet d'utiliser l'eau contenue dans la biocellulose humide pour dissoudre au moins partiellement la composition cosmétique d'une manière rapide, propre et ludique.

Une forme pulvérulente à faible teneur en eau voire complètement anhydre, peut faciliter la conservation de la composition, même en l'absence de conservateurs.

Le substrat peut être avantageusement contenu dans un conditionnement stérile, notamment sous forme humide. Lorsqu'il est sec, la conservation du substrat peut être facilitée.

La composition cosmétique pulvérulente peut être contenue dans tout conditionnement approprié, convenant à un usage unique ou multiple, fermant de manière étanche ou non, par exemple un sachet ou un flacon, éventuellement muni d'un organe de dosage et/ou de distribution, par exemple un tamis.

Lorsqu'elle est présente, la solution aqueuse de réhydratation du substrat peut également être contenue dans tout conditionnement approprié, convenant à un usage unique ou multiple, fermant de manière étanche ou non, par exemple un sachet, un flacon, une ampoule, éventuellement muni d'un organe de dosage et/ou de distribution.

Le cas échéant, la composition pulvérulente et la solution aqueuse peuvent être contenues dans deux compartiments d'un même conditionnement.

L'invention a encore pour objet, selon un autre de ses aspects, un procédé de préparation, notamment extemporanée, d'une composition cosmétique, comportant les étapes consistant à :
- mettre au contact d'un substrat humide comportant de la biocellulose une composition cosmétique pulvérulente au moins partiellement hydrosoluble, de manière à amener cette composition cosmétique pulvérulente à se dissoudre au moins partiellement au contact du substrat.

Le substrat peut être sec et réhydraté extemporanément. En variante, le substrat est déjà humide.

L'invention a encore pour objet un procédé de traitement cosmétique non thérapeutique au moyen d'une composition préparée comme ci-dessus.

La composition cosmétique pulvérulente peut être déposée sur le substrat puis celui-ci être appliqué sur des matières kératiniques. En variante, la composition cosmétique pulvérulente peut être appliquée sur les matières kératiniques, puis recouverte par le substrat.

Le substrat peut être laissé en place sur les matières kératiniques pendant une durée comprise entre 5 mn et 12 h par exemple.

### Substrat

Le substrat comporte de la biocellulose.

La biocellulose est un matériau obtenu par fermentation aérobie en milieu aqueux nutritif, de bactéries du genre Acetobacter (encore appelé Gluconacetobacter) (Z. Gromet-Elhanan, S. Hestrin, Synthesis of cellulose by Acetobacter Xylinum, J. Bacteriol. 85, 284-292, 1963 ; US 5 962 277).

La principale espèce utilisée est Acetobacter xylinum, même si d'autres peuvent également produire de la biocellulose, par exemple Acetobacter pasteurianus.

Les conditions de culture de cette bactérie pour produire la biocellulose sont parfaitement connues, notamment de la publication *Factors affecting the yield and properties of bacterial cellulose,* A. Krystynowicz et al., J. Indus. Microbiol. Biotech. 29, 189-195, 2002.

La biocellulose peut être utilisée sous forme pure ou sous une forme combinée à d'autres types de fibres, par exemple des fibres d'origine naturelle, par exemple des fibres issues du maïs, des fibres de chanvre, de lin, de coton, de jute, de kénaf, de raphia, de ramie, de Paja Toquilla, de sisal, de jonc, d'alfa, de phormium, de coco, de laine, de soie, de soja, d'abaca, de kumazasa, de plaqueminier, de kapok, de bardane, de céréales ou de bambou.

Les fibres de biocellulose peuvent être libres ou liées entre elles et/ou liées à d'autres fibres.

La biocellulose peut être utilisée en feuille, obtenue par exemple en compactant une culture de fibres de biocellulose, après rinçage de celles-ci. L'épaisseur de la feuille est par exemple comprise entre 50 et 4000 µm.

Préférentiellement, avant ajout de la composition pulvérulente, le substrat contient au moins 10% en poids de biocellulose, par rapport au poids total du substrat.

Le substrat à base de biocellulose peut contenir en outre, au moins au moment de l'utilisation, de 10 % à 99 % d'eau, préférentiellement de 40 % à 90 %.

Le substrat peut éventuellement être lié à un support, notamment un support permettant de le maintenir en place sur la peau.

Le substrat sous forme de feuille sèche ou humide peut avoir une forme de masque ou de patch adaptée à couvrir le visage, les yeux, le contour des yeux, les joues, les lèvres, les mains ou les ongles, entre autres.

Alternativement, le substrat sous forme de film peut être associé à un support souple dont le but est de permettre une application aisée de la composition sur la peau et les phanères, par exemple sous la forme d'un patch, d'un masque, d'une ceinture pour le corps ou d'un article vestimentaire.

Dans un mode préféré de réalisation, le substrat à base de biocellulose est stérilisé et conditionné de façon unitaire. La stérilisation peut être réalisée à l'aide de moyens connus par l'homme de l'art, tels que la stérilisation à la chaleur humide ou les rayonnements ionisants. En conséquence, aucun conservateur n'est nécessaire dans ce mode de réalisation.

Le substrat peut être conditionné à l'état sec en vue d'être réhydraté extemporanément avec de l'eau ou une solution aqueuse. Cette dernière est par exemple une eau thermale, minérale, une eau de plancton, une solution contenant au moins un actif, par exemple un actif incompatible avec un actif contenu dans la composition pulvérulente, c'est-à-dire ne pouvant être stocké à son contact sur une période prolongée sans subir de dégradation.

### Composition cosmétique pulvérulente

La composition cosmétique pulvérulente peut être sèche, c'est-à-dire contenant en masse moins de 10 % d'eau, préférentiellement moins de 5 %

La composition peut être sous forme de poudre libre hydrophile, que l'on applique extemporanément sur le substrat humide à base de biocellulose.

Dans des exemples de mise en oeuvre de l'invention, les particules de poudre peuvent se dissoudre en moins de 5 minutes, préférentiellement en moins de 2 minutes, sur le substrat humide. La dissolution de la poudre est définie par la perception visuelle de l'absence de particules solides résiduelles.

Le diamètre moyen des particules composant la poudre peut être compris par exemple entre 5 µm et 1000 µm, préférentiellement entre 50 µm et 500 µm

La composition peut contenir un ou plusieurs ingrédients cosmétiques actifs choisis parmi :
- les actifs sous forme de poudre tels que l'acide ascorbique, l'ascorbyl phosphate de sodium, l'ascorbyl phosphate de magnésium, le glucoside d'ascorbyl, l'acide hyaluronique, l'acide salicylique et ses dérivés, l'acide citrique, l'acide ellagique, l'acide kojique, l'acide férulique, les extraits végétaux sous forme de poudre, la dihydroxyacétone,
- les actifs sous forme solubilisée et adsorbée sur des particules solides.

Dans un mode de mise en oeuvre préféré de l'invention, la composition contient un ingrédient actif susceptible d'être facilement dégradé en milieu liquide et dont la stabilité physico-chimique est optimale lorsqu'il est sous forme de poudre.

On peut citer comme ingrédients actifs préférés, les vitamines, la dihydroxyacétone, l'acide férulique, les polyphénols, les extraits et hydrolysats végétaux, les extraits de souches microbiennes, les protéines, les peptides, les acides aminés, les bases puriques et pyrimidiques, les acides nucléiques, les oligonucléotides.

La composition peut en outre contenir des charges minérales ou organiques telles que les silices, les argiles, les talcs ou silicates de magnésium hydratés, les micas ou aluminosilicates, les argiles, le kaolin ou silicate d'aluminium hydraté, les nitrures de bore, les oxydes minéraux comme l'oxyde de zinc, les polymères et copolymères d'acide acrylique ou méthacrylique tels que les produits vendus sous le nom de "Polytrap" par la société DOW CORNING, des poudres de polyamide (Nylon^{®}).

La composition peut contenir des polymères hydrosolubles ou hydrodispersibles choisis parmi (1) les polymères de type protéique, tels que les protéines de blé ou de soja ; la kératine, par exemple les hydrolysats de kératine et les kératines sulfoniques ; la caséine ; l'albumine ; le collagène ; la gluteline ; le glucagon ; le gluten ; la zéine ; les gélatines et leurs dérivés ; (2) les polymères dérivant de la chitine ou du chitosane, tels que les polymères anioniques, cationiques, amphotères ou non ioniques de chitine ou de chitosane ; (3) les polymères polysaccharidiques tels que notamment (i) les polymères cellulosiques, comme l'hydroxyéthylcellulose, l'hydroxypropylcellulose, l'hydroxypropylméthylcellulose, la méthylcellulose, l'éthylhydroxyéthylcellulose, la carboxyméthylcellulose, et les dérivés quaternisés de la cellulose ; et (ii) les amidons et leurs dérivés ; (4) les polymères ou copolymères acryliques tels que les polyacrylates, les polyméthacrylates et leurs copolymères ; (5) les polymères vinyliques tels que les polyvinylpyrrolidones, les copolymères du méthylvinyléther et de l'anhydride maléique, le copolymère de l'acétate de vinyle et de l'acide crotonique, les copolymères de la vinylpyrrolidone et de l'acétate de vinyle, les copolymères de la vinylpyrrolidone et du caprolactame, les alcools polyvinyliques ; (6) les polymères d'origine naturelle, éventuellement modifiés, tels que la gomme arabique, la gomme de guar, les dérivés du xanthane, la gomme de karaya ; les alginates, les carraghénanes, les ulvanes et autres colloïdes algaux ; les glycoaminoglycanes, l'acide hyaluronique et ses dérivés ; la gomme laque, la gomme sandaraque, les dammars, l'élémis, les copals ; l'acide désoxyribonucléique ; les mucopolysaccharides, tels que l'acide hyaluronique, le sulfate de chondroïtine ; et les mélanges de ces polymères.

La composition peut aussi contenir un ou plusieurs sels choisi parmi les sels de calcium, de magnésium, de sodium, de potassium et leurs mélanges, et plus particulièrement le chlorure de magnésium, le chlorure de potassium, le chlorure de sodium, le chlorure de calcium, le bromure de magnésium, le bicarbonate de sodium, le phosphate de magnésium, le phosphate de sodium, le phosphate de potassium, le sulfate de calcium, le sulfate de magnésium et leurs mélanges.

La composition à appliquer sur le substrat humide à base de biocellulose peut être une poudre de nature effervescente.

La composition peut être obtenue par la mise en oeuvre d'un ou plusieurs moyens connus de l'homme de l'art tel que le broyage, la pulvérisation, le tamisage, le mélange, la granulation sèche ou humide, l'atomisation, la lyophilisation.

### Modes de présentation

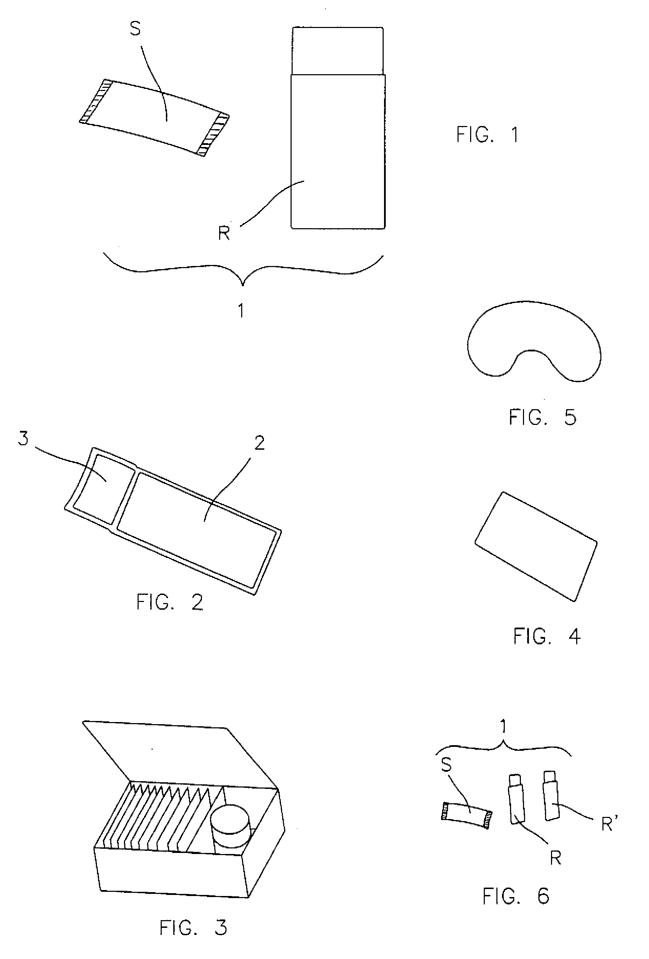

La composition peut être présentée sous forme de sachet ou contenue dans un récipient R permettant une distribution aisée et homogène sur le substrat humide, tandis que le substrat peut être contenu à l'état stérile dans un sachet S, comme illustré à la figure 1, afin de former un ensemble 1 selon l'invention.

Le cas échéant, le substrat et la composition peuvent être conditionnés dans deux compartiments séparés 2, 3 d'un sachet, comme illustré à la figure 2.

Plusieurs sachets S comportant chacun un substrat à usage unique peuvent être proposés dans un coffret 4 à l'utilisateur avec au moins un récipient R contenant la composition, comme illustré à la figure 3.

Le substrat peut être réalisé avec toute forme adaptée à la région à traiter, par exemple rectangulaire comme illustré à la figure 4 ou réniforme comme illustré à la figure 5, par exemple pour le traitement de la région du contour de l'oeil.

L'ensemble peut être proposé à l'utilisateur avec le substrat à l'état sec, la composition pulvérulente et une solution aqueuse pour réhydrater le substrat.

Cette solution aqueuse est par exemple contenue dans un récipient R', comme illustré à la figure 6, le récipient R' pouvant être proposé à l'utilisateur dans un même emballage que le substrat et la composition pulvérulente.

### EXEMPLES PROPOSES

### Exemples comparatifs

Une feuille de biocellulose est obtenue par fermentation statique en milieu aérobie d'une souche *d'Acetobacter Xylinum* (souche ATCC 23767) selon le procédé décrit dans Factors affecting the yield and properties of bacterial cellulose, A. Krystynowicz et al., J. Indus. Microbiol. Biotech. 29, 189-195, 2002.

La plaque de biocellulose obtenue après fermentation est rincée avec de l'eau distillée, lavée par une solution de soude diluée puis neutralisée par une solution d'acide acétique. Après rinçage avec de l'eau distillée, la plaque de biocellulose se présente sous forme d'une feuille d'environ 2 mm d'épaisseur. La biocellulose subit alors une stérilisation à la chaleur humide en autoclave à 121°C pendant 20 minutes.

5 g d'une poudre composée d'alginate de sodium, de sulfate de calcium, de phosphate de potassium et de silice sont déposés à la surface soit d'une feuille de biocellulose soit d'un hydrogel à base d'acide polyacrylique.

On note la rapidité avec laquelle la poudre se mouille et le temps après lequel aucune particule de poudre n'est plus visible.

On obtient les résultats suivants :

| | Temps de mouillage de la poudre | Temps après lequel aucune particule de poudre n'est plus visible |
|---|---|---|
| BIOCELLULOSE | Immédiat | 45 sec |
| HYDROGEL ACRYLIQUE > | 2 min | > 2 min |

On constate donc que le biocellulose permet une dissolution de la poudre presque instantanée, beaucoup plus rapide qu'un hydrogel.

### Exemple 1 Masque pour soin du visage pour un effet exfoliant et éclaircissant.

Un masque en biocellulose est obtenu par découpage à l'emporte-pièce d'une feuille de biocellulose. On répartit extemporanément et de manière homogène sur le masque une poudre composée de 0,05 g d'acide salicylique, 0,5 g d'acide ascorbique, 4 g d'acide citrique monohydraté et 5 g de bicarbonate de sodium, et l'on applique immédiatement le masque sur le visage pendant une période de 15 minutes.

### Exemple 2 Masque chauffant purifiant à l'argile

Un masque en biocellulose est obtenu par découpage à l'emporte-pièce d'une feuille de biocellulose.

On répartit extemporanément de façon homogène sur le masque une poudre composée de 4 g d'argile verte, de 0,5 g de kaolin et 2 g de sulfate de magnésium anhydre.

On applique immédiatement le masque sur le visage pendant une période de 15 minutes.

### Exemple 3 Patch anti-rides

Une plaque de 15 cm² de biocellulose obtenue par fermentation *d'Acetobacter Xylinum* est séchée pendant 24 h à 40 °C pour obtenir une feuille sèche.

D'autre part, une souche de plancton thermal *Vitreoscilla Filiformis* est cultivée à 26 °C pendant 48 h selon les conditions décrites dans la publication WO 94/02158.

La biomasse est séparée du milieu de culture par centrifugation, comme décrit dans US 6 242 229.

Le milieu de culture (A), encore appelé eau de plancton, est stérilisé à 121 °C pendant 20 mn. La biomasse (B) est transformée sous forme de poudre lyophilisée après congélation et sublimation.

La feuille sèche de biocellulose est hydratée extemporanément par 2 mL de milieu de culture (A) stérile puis 0,1 g de biomasse lyophilisée (B) est appliquée sur la feuille de biocellulose hydratée.

Le patch de biocellulose contenant le plancton thermal ainsi reconstitué extemporanément est alors appliqué sur le visage pour un effet anti-rides.

Bien entendu, l'invention n'est pas limitée aux exemples illustrés.

L'expression « comportant un » doit être comprise comme étant synonyme de « comportant au moins un », sauf si le contraire est spécifié.

## Revendications

1. Ensemble (1) comportant:
- un substrat comportant de la biocellulose,
- une composition cosmétique pulvérulente au moins partiellement hydrosoluble à mettre en contact avec le substrat.

2. Ensemble selon la revendication 1, la solution aqueuse comportant au moins un actif incompatible avec au moins un actif contenu dans la composition.

3. Ensemble selon la revendication 1, la composition cosmétique pulvérulente ayant une granulométrie moyenne allant de 5 µm à 1000 µm

4. Ensemble selon l'une quelconque des revendications précédentes, la composition cosmétique pulvérulente contenant au moins un actif choisi parmi les actifs sous forme de poudre tels que les vitamines, l'acide ascorbique, l'ascorbyl phosphate de sodium, l'ascorbyl phosphate de magnésium, le glucoside d'ascorbyl, l'acide hyaluronique, l'acide salicylique et ses dérivés, l'acide citrique, l'acide ellagique, l'acide kojique, l'acide férulique, les extraits et hydrolysats végétaux sous forme de poudre, la dihydroxyacétone, les polyphénols, les extraits de souches microbiennes, les protéines, les peptides, les acides aminés, les bases puriques et pyrimidiques, les acides nucléiques, les oligonucléotides, et leurs mélanges.

5. Ensemble selon l'une quelconque des revendications précédentes, la composition cosmétique pulvérulente comportant au moins un polymère hydrosoluble ou hydrodispersible.

6. Ensemble selon l'une quelconque des revendications précédentes, la composition cosmétique pulvérulente comportant au moins un composé pouvant donner lieu à une effervescence au contact de la biocellulose.

7. Ensemble selon l'une quelconque des revendications 1 à 6, la composition cosmétique pulvérulente étant sans conservateur.

8. Ensemble selon l'une quelconque des revendications précédentes, le substrat comportant au moins 10 % en masse de biocellulose.

9. Ensemble selon l'une quelconque des revendications précédentes, le substrat étant constitué de biocellulose humide.

10. Procédé de préparation d'une composition cosmétique, comportant les étapes consistant à :
- mettre au contact d'un substrat humide comportant de la biocellulose une composition cosmétique pulvérulente au moins partiellement hydrosoluble, de manière à amener cette composition cosmétique pulvérulente à se dissoudre au moins partiellement au contact du substrat.

11. Procédé selon la revendication 10, la mise en contact étant extemporanée.

12. Procédé selon la revendication 10 ou 11, le substrat étant humidifié extemporanément à partir d'eau ou d'une solution aqueuse.

13. Procédé de traitement cosmétique au moyen d'une composition préparée selon le procédé défini à l'une des revendications 10 à 12, la composition cosmétique étant appliquée sur des matières kératiniques.

14. Procédé selon la revendication 13, la composition étant d'abord déposée à l'état pulvérulent sur le substrat avant d'être appliquée sur les matières kératiniques.

15. Procédé selon l'une quelconque des revendications 13 à 14, le substrat étant laissé en place sur les matières kératiniques pendant une durée comprise entre 5 mn et 12 h.
